# EUROPEAN PATENT APPLICATION

(11) **EP 4 759 921 A1**
(43) Date of publication of application: **17.06.2026**
(21) Application number: 24851899.5
(22) Date of filing: 08.08.2024
(51) Int. Cl.: C12N 1/16, C12P 7/64

(54) **YEAST MUTANT STRAIN THAT PRODUCES OIL/FAT AND ALLOWS CELL CYCLE SYNCHRONIZATION**

(30) Priority: 08.08.2023 JP 2023129033
(71) Applicant: Teikyo University, Tokyo 173-8605 (JP)
(72) Inventor: TAKAYAMA, Yuko, Utsunomiya-shi, Tochigi 320-8551 (JP)
(74) Representative: Cohausz & Florack
(86) International application number: PCT/JP2024/028357
(87) International publication number: WO 2025/033481

(57) **Abstract**

Provided are oleaginous yeast mutant strains capable of being synchronized and arrested, by temperature change, in a specific cell cycle phase with high efficiency in lipid production. The mutant strain is a *Lipomyces starkeyi* strain that is synchronized and arrested in G1 phase by pre-culture at 23 to 26°C followed by main culture at 32 to 35°C, or *Lipomyces starkeyi* strains that are synchronized and arrested in G2/M phase by pre-culture at 20 to 23°C followed by main culture at 30 to 32°C.

## Description

### TECHNICAL FIELD

The present invention relates to an oleaginous yeast mutant strain capable of synchronizing the cell cycle.

### BACKGROUND ART

Since genus *Lipomyces*, oleaginous yeasts, accumulate approximately 70% lipids per cell, and their lipid constituent is similar to palm oil, it is expected to be utilized not only as a biofuel, but also as an alternative oil for food and cosmetics, leading to rapid advances in research.

The inventor, in advancing the elucidation of the molecular mechanisms of lipid production in *Lipomyces*, found that synchronization of the cells at the G1 phase of the cell cycle increases lipid accumulation by 1.4-fold (Non-patent literature 1).

However, the demand for lipid-production microorganisms and methods with high lipid production efficiency remains unchanged.

### CITATION LIST

### NON-PATENT LITERATURE

[Non-patent literature 1] Morimoto Y. et al., Journal of Cell Science (2022) 135 (16), jcs259996

### SUMMARY OF INVENTION

### TECHNICAL PROBLEM

An object of the present invention aims to provide oleaginous yeast mutant strains capable of synchronized and arrested in a specific cell cycle phase by temperature change, with high efficiency in lipid production.

### SOLUTION TO PROBLEM

The object can be solved by the following present inventions:
[1] A *Lipomyces starkeyi* strain that is synchronized and arrested in G1 phase by pre-culture at 23 to 26°C followed by main culture at 32 to 35°C.
[2] The *Lipomyces starkeyi* strain of [1], wherein an accession number is NITE BP-03941.
[3] A *Lipomyces starkeyi* strain that is synchronized and arrested in G2/M phase by pre-culture at 20 to 23°C followed by main culture at 30 to 32°C.
[4] The *Lipomyces starkeyi* strain of [3], wherein an accession number is NITE BP-03942 or NITE BP-03943.
[5] A method for inducing lipid accumulation by using the strain of any one of [1] to [4].
[6] A method for inducing lipid accumulation, comprising:
   pre-culturing the strain according to any one of [1] to [4] at 20 to 26°C, and subsequently culturing it at 30 to 35°C.

### ADVANTAGEOUS EFFECTS OF INVENTION

According to the present invention, without requiring expensive chemical treatments or specialized equipment, the mutant strains of the present invention can be synchronized and arrested in a specific cell cycle phase with high efficiency in lipid production by temperature change alone. By utilizing these mutant strains, increased lipid accumulation can be achieved.

### BRIEF DESCRIPTION OF THE DRAWINGS

[Fig. 1] Figure 1 shows the results of intracellular triacylglycerol quantification in *Lipomyces starkeyi* synchronized in the cell cycle by chemical treatments performed in the Referential Example below.
[Fig. 2] Figure 2 is a histogram showing the results of intracellular DNA contents of the Ls100, Ls76, Ls63, and Ls93 strains, measured by FACS analysis in Example 2 below.
[Fig. 3] Figure 3 is a graph showing the results of intracellular triacylglycerol quantification for the Ls100, Ls76, Ls63, and Ls93 strains in Example 3 below.

### DESCRIPTION OF EMBODIMENTS

### (Oleaginous yeast mutant strain of the present invention)

The oleaginous yeast mutant strains of the present invention are mutant strains of *Lipomyces starkeyi*. By pre-culture at normal temperature followed by culture at a temperature higher than normal, the cells can be synchronized and arrested in a specific cell cycle phase.

The first oleaginous yeast mutant strain of the present invention can be synchronized and arrested in G1 phase by performing pre-culture at 23 to 26°C followed by raising the temperature to 32 to 35°C for main culture.

The second oleaginous yeast mutant strains of the present invention can be synchronized and arrested in G2/M phase by performing pre-culture at 20 to 23°C followed by raising the temperature to 30 to 35°C for main culture.

The first oleaginous yeast mutant strain of the present invention includes a *Lipomyces starkeyi* Ls76 strain (accession number NITE BP-03941). The second oleaginous yeast mutant strains of the present invention include a *Lipomyces starkeyi* Ls63 strain (accession number NITE BP-03942) and a *Lipomyces starkeyi* Ls93 strain (accession number NITE BP-03943). These mutant strains exhibit higher lipid accumulation than that of known genus *Lipomyces*, and can be used as efficient lipid production yeasts. Mycological characteristics of the Ls76, Ls63, and Ls93 strains are that they are budding yeasts which proliferate well at 23°C but cease proliferation when the temperature exceeds 30°C.

### (Method for inducing lipid accumulation of the present invention)

The method for inducing lipid accumulation of the present invention can be carried out according to known methods for inducing lipid accumulation, except that the oleaginous yeast mutant strain of the present invention is used, and that the mutant strain is cultured under conditions that allow synchronized arrest in a desired cell cycle phase (specifically G1 phase or G2/M phase).

The method for inducing lipid accumulation of the present invention may be carried out, for example, by pre-culturing the strain prior to the main culture, and by inoculating the prepared culture into an appropriate medium for inducing lipid accumulation to perform main culture. The main culture may be carried out at a temperature of 20 to 35°C, and for approximately 1 to 7 days under shaking conditions.

In the method for inducing lipid accumulation using the first oleaginous yeast mutant strain of the present invention, the cells can be synchronized and arrested in G1 phase by pre-culture at 23 to 26°C followed by main culture at 32 to 35°C.

In the method for inducing lipid accumulation using the second oleaginous yeast mutant strains of the present invention, the cells can be synchronized and arrested in G2/M phase by pre-culture at 20 to 23°C followed by main culture at 30 to 32°C.

The pre-culture may be carried out using, for example, YPD medium.

In the present invention, lipid accumulation in oleaginous yeasts can be confirmed and quantified using known methods. Examples of such methods include a method in which after lipids are stained with a stain solution such as Oil Red, yeasts are photographed under a microscope and the lipids are visually confirmed; and a method in which a cell extract is prepared by dissolving or physically disrupting cell walls, and lipid components present in the extract are separated and qualified.

### EXAMPLES

The present invention will now be further illustrated by, but is by no means limited to, the following Examples.

### <<Referential Example: Measurement of lipid accumulation due to synchronization at each cell cycle phase>>

Synchronizing the cell cycle of *Lipomyces starkeyi* required expensive chemical treatments or specialized equipment. In this Referential Example, a *Lipomyces starkeyi* NBRC1289 strain (obtained from NBRP (National BioResource Project)), which was a wild-type strain isolated from soil was used, and the amounts of triacylglycerol (TAG) (unit: mg/10⁸ cells) in *Lipomyces* synchronized at each cell cycle phase were measured.

### (Synchronized arrest in S phase or synchronized arrest in G2/M phase)

Cells were pre-cultured at 26°C in YPD medium (2% D-glucose, 1% yeast extract, 2% hipolypeptone). Hydroxyurea (HU) or nocodazole (Noc) was added, and cells were cultured for 3.5 hours, and then harvested. As described below, hydroxyurea can synchronize and arrest cells in S phase, and nocodazole can synchronize and arrest cells at G2/M phase. After washing three times with sterile water, cells were transferred to N medium (0.3% sucrose, 0.025% KH₂PO₄, 0.066% MgSO₄·7H₂O, trace element solution (FeCl₃·6H₂O 1.7 mg/L, MnSO₄·6H₂O 0.51 mg/L, ZnSO₄·7H₂O 4.5 mg/L)) at a cell concentration of approximately 3 × 10⁶ cells/mL, and cultured at 26°C for 18 hours. After 5 × 10⁷ cells were harvested, the amount of triacylglycerol was quantified.

### (Synchronized arrest in G1 phase)

Cells were pre-cultured at 26°C in YPD medium. Nocodazole (Noc) was added, and cells were cultured for 3 hours, and then harvested. After washing three times with sterile water, cells were transferred to YPD medium and cultured for 30 minutes. Rapamycin (Rapa) was added, and cells were further cultured for 2.5 hours. As described below, rapamycin can synchronize and arrest cells in G1 phase. After washing three times with sterile water, cells were transferred to N medium at a cell concentration of approximately 3 × 10⁶ cells/mL, and cultured at 26°C for 18 hours. After 5 × 10⁷ cells were harvested, the amount of triacylglycerol was quantified.

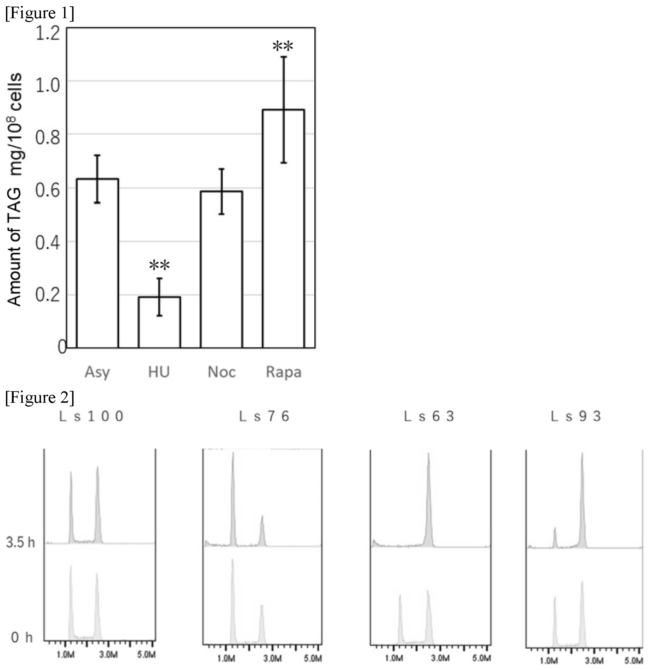

The results are shown in Figure 1.

In Figure 1, Asy denotes asynchronous, i.e., N medium, HU denotes hydroxyurea, Noc denotes nocodazole, and Rapa denotes rapamycin.

Cells treated with hydroxyurea (HU) were synchronized and arrested in S phase, cells treated with nocodazole (Noc) were synchronized and arrested in G2/M phase, and cells treated with rapamycin (Rapa) were synchronized and arrested in G1 phase.

The error bars (mean ± standard deviation) in Figure 1 were calculated from independent cell cultures [n = 16, 10, 14, 20 (from the left of the graph; number of experiments)]. The symbol "**" in Figure 1 indicates P<0.001 (compared to N medium; Dunnett's multiple comparison test).

The highest triacylglycerol accumulation was observed when cells were synchronized and arrested in G1 phase (approximately 1.4-fold).

### <<Example 1: Isolation and identification of oleaginous yeast mutant strains>>

In this Example, we used a *Lipomyces starkeyi* Ls100 strain (deposited with NBRP), a derivative of a *Lipomyces starkeyi* NBRC1289 strain. After ultraviolet irradiation, colonies were inoculated to YPD agar plates, and incubated under high-temperature conditions (30°C and 32°C). We confirmed whether strains that exhibited poor growth under high-temperature conditions would again show poor growth under high-temperature conditions. We cultured strains exhibiting poor growth under high-temperature conditions, and screened them by FACS analysis using the criterion of whether cells arrested in a specific cell cycle phase. As a result, we successfully obtained oleaginous yeast mutant strains that arrested the cell cycle under high-temperature conditions. More specifically, we obtained one strain (designated Ls76) that synchronized and arrested in G1 phase and two strains (designated Ls63 and Ls93) that synchronized and arrested in G2/M phase. In summary, approximately 100 strains exhibiting poor growth under high-temperature conditions were isolated from approximately 1,700 colonies that formed after UV irradiation, and ultimately, three strains that synchronized and arrested the cell cycle were obtained.

### <<Example 2: Synchronized arrest of cell cycle in oleaginous yeast mutant strains>> (Ls76 strain that synchronizes and arrests in G1 phase)

Cells were pre-cultured at 26°C in YPD medium, then cultured for 3.5 hours after shifting the temperature to 32°C, and harvested. After washing three times with sterile water, cells were inoculated to N medium at a cell concentration of approximately 3 × 10⁶ cells/mL, and culture was initiated at 26°C or 32°C. Every other day, 5 × 10⁷ cells were harvested, and intracellular DNA content and the amount of triacylglycerol were quantified.

### (Ls63 strain and Ls93 strain that synchronize and arrest in G2/M phase)

Cells were pre-cultured at 23°C in YPD medium, then cultured for 3.5 hours after shifting the temperature to 30°C, and harvested. After washing three times with sterile water, cells were inoculated to N medium at a cell concentration of approximately 3 × 10⁶ cells/mL, and culture was initiated at 26°C or 30°C. Every other day, 5 × 10⁷ cells were harvested, and intracellular DNA content and the amount of triacylglycerol were quantified.

Figure 2 shows the results of measuring intracellular DNA content by FACS (fluorescence-activated cell sorting) analysis.

For each column, the lower row shows DNA content at the start of 32°C culture after changing from 26°C culture to the 32°C culture, while the upper row shows DNA content 3.5 hours after the start of the 32°C culture.

For example, among the two peaks in the upper row of the Ls100 strain, the left peak represents the state before DNA replication begins, while the right peak represents the state after DNA synthesis has finished (i.e., a twofold increase in DNA content).

The obtained mutant strains exhibited the following characteristics:
Ls76 strain (accession number NITE BP-03941): The cells can be synchronized and arrested in G1 phase by pre-culture at 26°C followed by main culture at 32°C for 3.5 hours.
Ls63 strain (accession number NITE BP-03942): The cells can be synchronized and arrested in G2/M(1) phase by pre-culture at 23°C followed by main culture at 30°C for 3.5 hours.
Ls93 strain (accession number NITE BP-03943): The cells can be synchronized and arrested in G2/M(2) phase by pre-culture at 23°C followed by main culture at 30°C for 3.5 hours.
G2/M phase-arrested cells of the Ls63 strain or the Ls93 strain can restart the cell cycle (reversible strains) when returned to a low temperature, and therefore, these strains have even greater value from a cell biological perspective.

### <<Example 3: Time courses of intracellular lipid accumulation in oleaginous yeast mutant strains>>

In this Example, mutant strains, the *Lipomyces starkeyi* Ls76 strain, Ls63 strain, and Ls93 strain, were used. We observed the time courses of intracellular DNA content and triacylglycerol accumulation in these mutant strains under conditions where cell proliferation was arrested in G1 phase or G2/M phase by changing the medium and shifting the culture temperature.

For the Ls76 strain, cells were pre-cultured at 26°C in YPD medium, then cultured for 3.5 hours after shifting the temperature to 32°C, and harvested. After washing three times with sterile water, cells were inoculated to N medium at a cell concentration of approximately 3 × 10⁶ cells/mL, and culture was initiated at 26°C or 32°C. After 1 day and 4 days of culture, 5 × 10⁷ cells were harvested, and intracellular DNA content and the amount of triacylglycerol were quantified.

For the Ls63 strain and the Ls93 strain, cells were pre-cultured at 23°C in YPD medium, then cultured for 3.5 hours after shifting the temperature to 30°C, and harvested. After washing three times with sterile water, cells were inoculated to N medium at a cell concentration of approximately 3 × 10⁶ cells/mL, and culture was initiated at 23°C or 30°C. After 1 day and 4 days of culture, 5 × 10⁷ cells were harvested, and intracellular DNA content and the amount of triacylglycerol were quantified.

The Ls100 strain, the parent strain of these temperature-sensitive mutants, was used as a control in the same experiment.

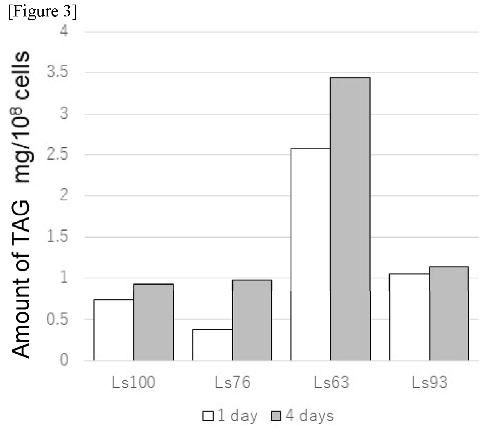

Figure 3 shows the amounts of intracellular triacylglycerol in the Ls100, Ls76, Ls63, and Ls93 strains after 1 and 4 days of culture. The Ls63 strain exhibited extremely remarkable lipid accumulation.

According to the oleaginous yeast mutants of the present invention, without requiring expensive chemical treatments or specialized equipment, the mutant strains of the present invention can be synchronized and arrested in a specific cell cycle phase with high efficiency in lipid production by temperature change alone. By utilizing these mutant strains, increased lipid accumulation can be achieved.

### INDUSTRIAL APPLICABILITY

The oleaginous yeast mutant strain of the present invention can be utilized in the field of lipid production using yeasts.

### REFERENCE TO DEPOSITED BIOLOGICAL MATERIAL

*Lipomyces starkeyi* Ls76 was deposited internationally at the NITE Patent Microorganisms Depositary (NPMD), National Institute of Technology and Evaluation (Address: #122, 2-5-8 Kazusakamatari, Kisarazu-shi, Chiba 292-0818, Japan) on July 11, 2023 under accession number NITE BP-03941.

*Lipomyces starkeyi* Ls63 was deposited internationally at the NITE Patent Microorganisms Depositary (NPMD), National Institute of Technology and Evaluation (Address: #122, 2-5-8 Kazusakamatari, Kisarazu-shi, Chiba 292-0818, Japan) on July 11, 2023 under accession number NITE BP-03942.

*Lipomyces starkeyi* Ls93 was deposited internationally at the NITE Patent Microorganisms Depositary (NPMD), National Institute of Technology and Evaluation (Address: #122, 2-5-8 Kazusakamatari, Kisarazu-shi, Chiba 292-0818, Japan) on July 11, 2023 under accession number NITE BP-03943.

## Claims

1. A *Lipomyces starkeyi* strain that is synchronized and arrested in G1 phase by pre-culture at 23 to 26°C followed by main culture at 32 to 35°C.

2. The *Lipomyces starkeyi* strain according to claim 1, wherein an accession number is NITE BP-03941.

3. A *Lipomyces starkeyi* strain that is synchronized and arrested in G2/M phase by pre-culture at 20 to 23°C followed by main culture at 30 to 32°C.

4. The *Lipomyces starkeyi* strain according to claim 3, wherein an accession number is NITE BP-03942 or NITE BP-03943.

5. A method for inducing lipid accumulation by using the strain according to any one of claims 1 to 4.

6. A method for inducing lipid accumulation, comprising:
pre-culturing the strain according to any one of claims 1 to 4 at 20 to 26°C, and
subsequently culturing it at 30 to 35°C.
